(19)

Europäisches Patentamt
European Patent Office
Office européen des brevets

(11) **EP 4 492 056 A1**

(12) **EUROPEAN PATENT APPLICATION**
published in accordance with Art. 153(4) EPC

(43) Date of publication:
**15.01.2025 Bulletin 2025/03**

(21) Application number: **23811450.8**

(22) Date of filing: **30.03.2023**

(51) International Patent Classification (IPC):
**G01N 33/2028** (2019.01)    **G01N 21/17** (2006.01)

(52) Cooperative Patent Classification (CPC):
**G01N 21/17; G01N 33/2028; G06T 7/00**

(86) International application number:
**PCT/JP2023/013409**

(87) International publication number:
**WO 2023/228571 (30.11.2023 Gazette 2023/48)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **23.05.2022 JP 2022084130**

(71) Applicant: **JFE Steel Corporation**
**Tokyo 100-0011 (JP)**

(72) Inventors:
• **KIYOKANE, Naoya**
  **Tokyo 100-0011 (JP)**
• **ISHIKAWA, Shin**
  **Tokyo 100-0011 (JP)**

(74) Representative: **Hoffmann Eitle**
**Patent- und Rechtsanwälte PartmbB**
**Arabellastraße 30**
**81925 München (DE)**

(54) **REPRESENTATIVE STRUCTURE PHOTOGRAPH DETERMINING METHOD, REPRESENTATIVE STRUCTURE PHOTOGRAPH DETERMINING DEVICE, IMAGING DEVICE, AND PROGRAM**

(57)      Provided are a representative micrograph determining method, a representative micrograph determining device, an imaging device, and a program that can objectively and efficiently determine a representative micrograph from a plurality of micrographs of a metal material. The representative micrograph determining method includes: an input process of acquiring a plurality of micrographs taken at two or more magnifications for two or more fields of view of a metal material (S11); a phase classification process of classifying phases in the plurality of micrographs (S12); a quantitative value calculation process of calculating quantitative values of the phases classified (S13); a magnification determination process of determining a representative magnification based on the quantitative values (S14); and an output process of outputting the representative magnification (S17).

*FIG. 2*

**Description**

TECHNICAL FIELD

The present disclosure relates to a representative micrograph determining method, a representative micrograph determining device, an imaging device, and a program. The present disclosure relates, in particular, to a representative micrograph determining method, a representative micrograph determining device, an imaging device, and a program, for determining a representative micrograph of micrographs of a metal material.

BACKGROUND

Typically, even when metal materials including steel have the same composition, properties are strongly dependent on metallic structure at the scale of an optical microscope or an electron microscope (for example, mm to $\mu$m scale).

For example, in the development of high strength steel sheets, methods to change the composition are used, such as solid solution strengthening by adding a solid-solution-strengthening element and strengthening by precipitation by adding a strengthening-by-precipitation element. Further, aside from such methods, other methods may be used to change the final metallic structure by changing heat treatment conditions with the same composition.

Thus, in the development of high strength steel sheets and the like, controlling not only the composition but also the metallic structure is important. Therefore, quantitatively evaluating metallic structure of a metal material by observation with an optical microscope or an electron microscope is important. For example, Patent Literature (PTL) 1 describes a method for preparing observation samples for observation with an electron microscope.

After carrying out sample preparation such as known polishing and etching methods on metal material such as a steel sheet, metallic structure can be observed using known imaging equipment such as an electron microscope, which typically allows classification of each phase because contrast is different for each phase. For example, regarding a steel material, when a representative steel sheet consisting of ferrite phase and pearlite phase is prepared as a sample by a known method and imaged by an optical microscope, ferrite phase is observed as having gray contrast and pearlite phase as having black contrast. Accordingly, ferrite phase and pearlite phase can be classified. Development is carried out by conducting observations of metallic structure using such methods and feeding back the results of the observations.

CITATION LIST

Patent Literature

PTL 1: JP 2009-287941 A

SUMMARY

(Technical Problem)

When a metal material is newly developed, appropriate magnification for observing the metallic structure is often unclear. In such a case, conventionally, magnification is determined and field of view is selected subjectively by an observer. Accordingly, inappropriate selection may lead to misinterpretation of correlation between metallic structure and material property. When a production process is determined based on a misinterpreted correlation, there is a risk of being unable to produce a product having a desired property. Further, when determining a representative micrograph, an observer may spend time comparing and selecting multiple fields of view while changing the magnification, which reduces work efficiency.

In view of such circumstances, it would be helpful to provide a representative micrograph determining method, a representative micrograph determining device, an imaging device, and a program that can objectively and efficiently determine a representative micrograph from a plurality of micrographs of a metal material.

(Solution to Problem)

(1) A representative micrograph determining method according to an embodiment of the present disclosure comprises:

an input process of acquiring a plurality of micrographs taken at two or more magnifications for two or more fields of view of a metal material;
a phase classification process of classifying phases in the plurality of micrographs;
a quantitative value calculation process of calculating quantitative values of the phases classified;
a magnification determination process of determining a representative magnification based on the quantitative values; and
an output process of outputting the representative magnification.

(2) A representative micrograph determining method according to an embodiment of the present disclosure comprises:

an input process of acquiring a plurality of micrographs taken at a defined magnification for two or more fields of view of a metal material;
a phase classification process of classifying phases in the plurality of micrographs;
a quantitative value calculation process of calculating quantitative values of the phases classified;
a deviation calculation process of calculating deviation of the quantitative values;
a selection process of selecting a representative micrograph from the plurality of micrographs based on the deviation; and
an output process of outputting the representative micrograph.

(3) A representative micrograph determining method according to an embodiment of the present disclosure comprises:

an input process of acquiring a plurality of micrographs taken at two or more magnifications for two or more fields of view of a metal material;
a phase classification process of classifying phases in the plurality of micrographs;
a quantitative value calculation process of calculating quantitative values of the phases classified;
a magnification determination process of determining a representative magnification based on the quantitative values; and
a deviation calculation process of calculating deviation of the quantitative values for a set of the plurality of micrographs corresponding to the representative magnification;
a selection process of selecting a representative micrograph from the set of the plurality of micrographs corresponding to the representative magnification based on the deviation; and
an output process of outputting the representative micrograph.

(4) The representative micrograph determining method according to any one of (1) to (3), as an embodiment of the present disclosure, wherein
the quantitative values are at least one of area fraction, ellipsoid size, Feret diameter, average diameter, circularity, or number density.

(5) A representative micrograph determining device according to an embodiment of the present disclosure comprises:

an input interface configured to acquire a plurality of micrographs taken at two or more magnifications for two or more fields of view of a metal material;
a phase classification unit configured to classify phases in the plurality of micrographs;
a quantitative value calculation unit configured to calculate quantitative values of the phases classified;
a magnification determination unit configured to determine a representative magnification based on the quantitative values; and
an output interface configured to output the representative magnification.

(6) A representative micrograph determining device according to an embodiment of the present disclosure comprises:

an input interface configured to acquire a plurality of micrographs taken at a defined magnification for two or more fields of view of a metal material;
a phase classification unit configured to classify phases in the plurality of micrographs;
a quantitative value calculation unit configured to calculate quantitative values of the phases classified;
a deviation calculation unit configured to calculate deviation of the quantitative values;
a selection unit configured to select a representative micrograph from the plurality of micrographs based on the deviation; and

an output interface configured to output the representative micrograph.

(7) A representative micrograph determining device according to an embodiment of the present disclosure comprises:

an input interface configured to acquire a plurality of micrographs taken at two or more magnifications for two or more fields of view of a metal material;
a phase classification unit configured to classify phases in the plurality of micrographs;
a quantitative value calculation unit configured to calculate quantitative values of the phases classified;
a magnification determination unit configured to determine a representative magnification based on the quantitative values; and
a deviation calculation unit configured to calculate deviation of the quantitative values for a set of the plurality of micrographs corresponding to the representative magnification;
a selection unit configured to select a representative micrograph from the set of the plurality of micrographs corresponding to the representative magnification based on the deviation; and
an output interface configured to output the representative micrograph.

(8) An imaging device according to an embodiment of the present disclosure, configured to
take the plurality of micrographs acquired by the representative micrograph determining device according to any one of (5) to (7).

(9) The imaging device according to (8), as an embodiment of the present disclosure, wherein
the imaging device is an optical microscope or a scanning electron microscope.

(10) A program according to an embodiment of the present disclosure

is configured to cause a computer to function as
an input interface configured to acquire a plurality of micrographs taken at two or more magnifications for two or more fields of view of a metal material;
a phase classification unit configured to classify phases in the plurality of micrographs;
a quantitative value calculation unit configured to calculate quantitative values of the phases classified;
a magnification determination unit configured to determine a representative magnification based on the quantitative values; and
an output interface configured to output the representative magnification.

(11) A program according to an embodiment of the present disclosure

is configured to cause a computer to function as
an input interface configured to acquire a plurality of micrographs taken at a defined magnification for two or more fields of view of a metal material;
a phase classification unit configured to classify phases in the plurality of micrographs;
a quantitative value calculation unit configured to calculate quantitative values of the phases classified;
a deviation calculation unit configured to calculate deviation of the quantitative values;
a selection unit configured to select a representative micrograph from the plurality of micrographs based on the deviation; and
an output interface configured to output the representative micrograph.

(12) A program according to an embodiment of the present disclosure

is configured to cause a computer to function as
an input interface configured to acquire a plurality of micrographs taken at two or more magnifications for two or more fields of view of a metal material;
a phase classification unit configured to classify phases in the plurality of micrographs;
a quantitative value calculation unit configured to calculate quantitative values of the phases classified;
a magnification determination unit configured to determine a representative magnification based on the quantitative values; and
a deviation calculation unit configured to calculate deviation of the quantitative values for a set of the plurality of micrographs corresponding to the representative magnification;
a selection unit configured to select a representative micrograph from the set of the plurality of micrographs corresponding to the representative magnification based on the deviation; and
an output interface configured to output the representative micrograph.

(Advantageous Effect)

According to the present disclosure, it is possible to provide a representative micrograph determining method, a representative micrograph determining device, an imaging device, and a program that can objectively and efficiently determine a representative micrograph from a plurality of micrographs of a metal material.

BRIEF DESCRIPTION OF THE DRAWINGS

In the accompanying drawings:

FIG. 1 is a schematic diagram illustrating an example configuration of a representative micrograph determining system including a representative micrograph determining device according to an embodiment of the present disclosure;
FIG. 2 is a flowchart illustrating processing of a representative micrograph determining method according to an embodiment of the present disclosure;
FIG. 3 is a schematic diagram illustrating an example configuration of a representative micrograph determining system including a representative micrograph determining device according to an embodiment of the present disclosure;
FIG. 4 is a flowchart illustrating processing of a representative micrograph determining method according to an embodiment of the present disclosure;
FIG. 5 is a schematic diagram illustrating an example configuration of a representative micrograph determining system including a representative micrograph determining device according to an embodiment of the present disclosure;
FIG. 6 is a flowchart illustrating processing of a representative micrograph determining method according to an embodiment of the present disclosure;
FIG. 7 illustrates micrographs used in an Example;
FIG. 8 illustrates the micrographs of FIG. 7 after a phase classification process; and
FIG. 9 illustrates a representative micrograph.

DETAILED DESCRIPTION

Hereinafter, with reference to the drawings, a representative micrograph determining method, a representative micrograph determining device, an imaging device, and a program are described according to embodiments of the present disclosure. In each drawing, identical or equivalent parts are marked with the same reference sign. In description of the following embodiments, description of identical or equivalent parts is omitted or simplified as appropriate.

[First embodiment]

(Representative micrograph determining system)

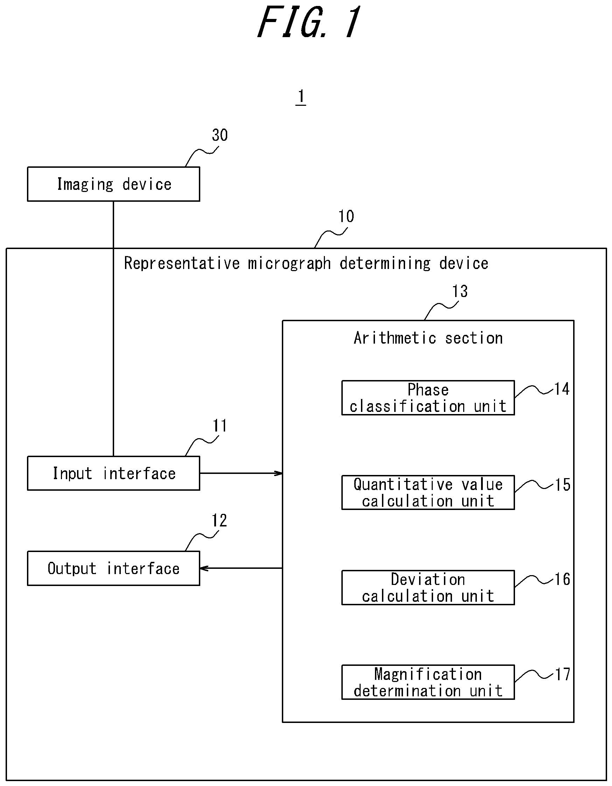

FIG. 1 is a block diagram of a representative micrograph determining system 1 including a representative micrograph determining device 10 according to a first embodiment. The representative micrograph determining system 1 includes the representative micrograph determining device 10 and an imaging device 30. The representative micrograph determining device 10 includes an input interface 11, an output interface 12, and an arithmetic section 13. The arithmetic section 13 includes a phase classification unit 14, a quantitative value calculation unit 15, and a magnification determination unit 17. The arithmetic section 13 may further include a deviation calculation unit 16, as in the present embodiment.

(Imaging device)

The imaging device 30 takes a plurality of micrographs of a metal material to be acquired by the representative micrograph determining device 10. The imaging device 30 may be, for example, an optical microscope or a scanning electron microscope, but is not limited to these examples and may be any device that has a function of imaging microstructure of a metal material.

(Representative micrograph determining device)

The representative micrograph determining device 10 executes processing for determining a representative micrograph from a plurality of micrographs of a metal material taken by the imaging device 30. Here, the metal material is a steel material or a metal material having a plurality of metallic phases. A representative micrograph is a micrograph that allows quantitative evaluation of metallic structure. The representative micrograph determining device 10 may be configured to directly determine a representative micrograph and may be configured to determine a property of a representative micrograph. The property of a representative micrograph to be determined may be, for example, magnification or scale. According to the present embodiment, the representative micrograph determining device 10 determines representative magnification (representative micrograph magnification).

(Input interface)

The input interface 11 is an input interface of the representative micrograph determining device 10, and acquires data required for processing determining a representative micrograph. According to the present embodiment, the input interface 11 acquires a plurality of micrographs taken at two or more magnifications for two or more fields of view of a metal material. The data format for the plurality of micrographs may be a commonly used image data format such as TIFF, BMP, or the like.

(Output interface)

The output interface 12 is an output interface of the representative micrograph determining device 10, and outputs a representative micrograph or representative micrograph property that is determined. The output interface 12 may transmit a representative micrograph or information on a representative micrograph property that is determined to another device or the like. Further, the output interface 12 may display a representative micrograph or a representative micrograph property that is determined on a display device such as any of various displays or the like. According to the present embodiment, the output interface 12 outputs a representative magnification.

(Arithmetic section)

The arithmetic section 13 performs calculations to determine a representative micrograph or a representative micrograph property. Further, the arithmetic section 13 may function as a controller that controls the representative micrograph determining device 10 as a whole. The arithmetic section 13 may be one or more processors. Each processor may be, but is not limited to, a general-purpose processor or a dedicated processor specialized for particular processing, and may be any processor.

As described above, according to the present embodiment, the arithmetic section 13 includes the phase classification unit 14, the quantitative value calculation unit 15, the deviation calculation unit 16, and the magnification determination unit 17. The functions of the phase classification unit 14, the quantitative value calculation unit 15, the deviation calculation unit 16, and the magnification determination unit 17 may be realized by software. For example, one or more programs may be stored in a storage device accessible by the arithmetic section 13. A program stored in the storage device, when read by the arithmetic section 13 that is a processor, may cause the arithmetic section 13 to function as the phase classification unit 14, the quantitative value calculation unit 15, the deviation calculation unit 16, and the magnification determination unit 17.

(Phase classification unit)

The phase classification unit 14 classifies phases in a plurality of micrographs. The details of processing executed by the phase classification unit 14 are described later.

(Quantitative value calculation unit)

The quantitative value calculation unit 15 calculates quantitative values for the phases classified by the phase classification unit 14. The details of processing executed by the quantitative value calculation unit 15 are described later.

(Magnification determination unit)

The magnification determination unit 17 determines a representative magnification based on quantitative values calculated by the quantitative value calculation unit 15. The details of processing executed by the magnification determination unit 17 are described later.

(Deviation calculation unit)

The deviation calculation unit 16 calculates deviation of quantitative values in order to determine a final representative magnification when there are a plurality of candidates for a representative magnification determined by the magnification determination unit 17. The details of processing executed by the deviation calculation unit 16 are described later.

Here, the representative micrograph determining device 10 is not limited to a specific device, but may be realized by a computer as an example. The computer may be a commercially available, general-purpose computer, for example. The computer may, for example, include a storage device, such as memory and a hard disk drive, a CPU, and input/output devices. The arithmetic section 13 may be realized by a CPU. The program to be read by the arithmetic section 13 may be stored in a storage device. The input interface 11 and the output interface 12 may be realized by input/output devices.

(Representative micrograph determining method)

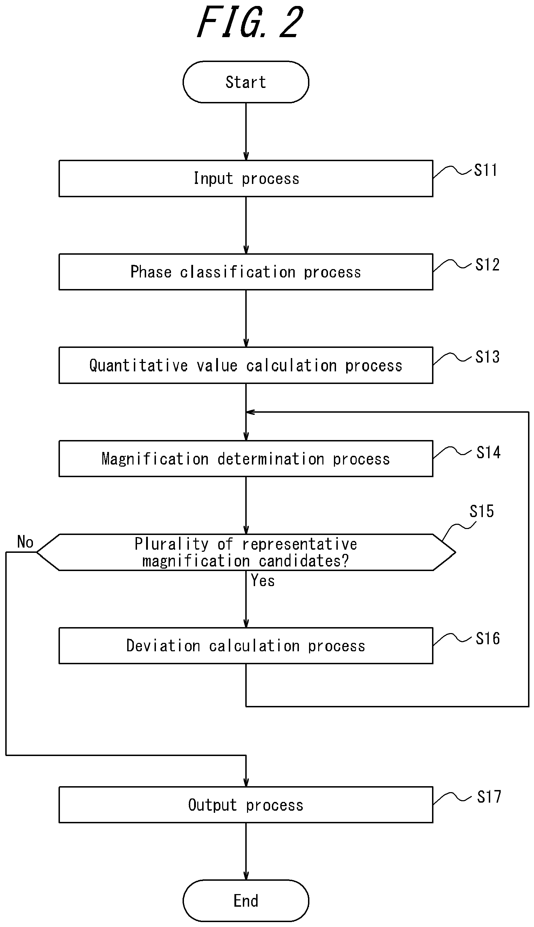

FIG. 2 is a flowchart illustrating processing of the representative micrograph determining method executed by the representative micrograph determining device 10 according to the present embodiment. In summary, the representative micrograph determining method includes, in this order: an input process of acquiring a plurality of micrographs taken at two or more magnifications for two or more fields of view of a metal material; a phase classification process of classifying phases in the plurality of micrographs; a quantitative value calculation process of calculating quantitative values of the phases classified; a magnification determination process of determining a representative magnification based on the quantitative values; and an output process of outputting the representative magnification. However, when there are a plurality of candidates for a representative magnification, an additional deviation calculation process is performed to calculate the deviation of quantitative values in order to determine a final representative magnification. Here, when a representative magnification could not be determined, the representative micrograph determining method may be executed again with an additional micrograph acquired in the input process.

(Input process)

The input process is a process in which the input interface 11 acquires a plurality of micrographs taken at two or more magnifications for two or more fields of view of a metal material (step S11).

After sample preparation of a surface of a metal material (sample) by a known method, such as polishing, a micrograph is preferably taken by a known imaging device such as an optical microscope or a scanning electron microscope. Sample preparation is typically carried out by rough polishing followed by finish polishing. Rough polishing and finish polishing may be carried out by known methods. Rough polishing by a known method may, for example, be polishing to remove scratches visible at the naked eye level, using a commercially available paper file coated with abrasive grains on paper (such as emery paper). Further, finish polishing by a known method is polishing carried out with a 0.05 $\mu$m to 2 $\mu$m abrasive until the sample has a mirror-like finish. As an abrasive, a known abrasive such as diamond, silica, or the like may be used. Mirror polishing is carried out until polishing scratches are no longer noticeable when observed under an optical microscope at 10$\times$ to 500$\times$ magnification. When many polishing scratches remain, they may cause errors in phase classification during the phase classification process, and therefore carrying out polishing with as few scratches as possible is desirable. After mirror polishing is carried out, etching with nital or the like may be carried out to clarify phase contrast. Etching may be omitted, such as for a sample that can be phase classified without etching.

The number of fields of view for the micrographs is two or more. The greater the number of fields of view, the less the variation in quantitative value of phases at each magnification. Three or more fields of view for the micrographs is preferred. Five or more fields of view for the micrographs is more preferable. The fields of view are preferably determined randomly. Further, two or more fields of view may be acquired by taking a series of images while changing the field of view slightly. Further, when the imaging device 30 includes a function that automatically determines the field of view, such as by using random numbers via a program, such a function may be used.

The number of levels of magnification of micrographs is 2 or more. The greater the number of levels of magnification, the more precise the relationship between acquired magnification and quantitative values of the phases for each magnification, and the easier it becomes to determine an appropriate magnification. The number of levels of magnification of micrographs is preferably 3 or more. The number of levels of magnification of micrographs is more preferably 5 or more. Magnification is preferably selected randomly. For example, when observing dual phase steel (DP steel) consisting of ferrite phase and martensite phase, magnification is preferably selected in a range from 500$\times$ to 3000$\times$. For example, when observing a coated or plated layer of a coated or plated steel sheet, magnification is preferably selected in a range from 1000$\times$ to 5000$\times$.

(Phase classification process)

The phase classification process is a process in which the phase classification unit 14 classifies phases in a plurality of micrographs (step S12).

The contrast of phases generally differ from each other, and therefore micrographs can be classified into phases. For example, phases are identified in a micrograph, and each phase is labeled with a different color or the like for classification. Phase classification may be performed by identifying phases with the naked eye and classifying by hand painting, by using binarization of image luminance values, or by advanced image interpretation methods. Application of a classification method that is as precise as possible is preferred.

However, phase classification by hand painting lacks objectivity. Therefore, binarization or advanced image interpretation methods are preferably used. As exemplified below, advanced image interpretation methods are more preferably used.

In an advanced image interpretation method, a plurality of regions of each phase are specified from a captured micrograph, feature values are calculated from image luminance values in the specified regions, and classification is performed using a method such as a random forest model, a neural network model, or a support vector machine that repeatedly converts calculated feature values into N values. One or more of the following eight specific examples (C1 to C8) is preferably used for feature values.

(C1: constant feature value)

A constant feature value is a feature value that indicates the luminance value of a micrograph itself.

(C2: mean feature value)

A mean feature value is a feature value that indicates the mean value of luminance values in a defined range of a micrograph. That is, a mean feature value is the result of taking a defined range "(number of pixels x) × (number of pixels y)" from each phase of a micrograph and averaging luminance values within the range. Here, "number of pixels x" and "number of pixels y" may be the same size or different sizes. The area of "(number of pixels x) × (number of pixels y)" need not be rectangular, but is preferably a circular area when a micrograph is circular in shape, and may be calculated for a plurality of numbers of pixels x and y. Lower limits of "number of pixels x" and "number of pixels y" are preferably, for example, in a range that includes a size greater than noise in a micrograph and includes a size less than 1/2 the smaller crystal grain size of the plurality of phases of the metallic structure. When upper limits are too large, the mean feature value may be affected by grain boundaries or other adjacent phases, and therefore are preferably in a range that includes a size less than 1/2 the crystal grain size of the larger crystal grain size.

Here, "noise in a micrograph" indicates, for example, a portion of a micrograph in which the luminance value suddenly increases. Further, the number of pixels x and y being greater than noise indicates making the numbers greater than noise width.

(C3: Gaussian feature value)

A Gaussian feature value is a feature value that indicates an average of luminance values in a defined range of a micrograph, with greater weight the closer to the center. That is, a Gaussian feature value is the result of taking a defined range "(number of pixels x) × (number of pixels y)" from each phase of a micrograph and taking averaged luminance values within the range while weighting central pixels more heavily. Here, "number of pixels x" and "number of pixels y" may be the same size or different sizes. The area of "(number of pixels x) × (number of pixels y)" need not be rectangular, but is preferably a circular area when a micrograph is circular in shape, and may be calculated for a plurality of numbers of pixels x and y. Lower limits of "number of pixels x" and "number of pixels y" are preferably, for example, in a range that includes a size greater than noise in a micrograph and includes a size less than 1/2 the smaller crystal grain size of the plurality of phases of the metallic structure. When upper limits are too large, the mean feature value may be affected by grain boundaries or other adjacent phases, and therefore are preferably in a range that includes a size less than 1/2 the crystal grain size of the larger crystal grain size.

(C4: median feature value)

A median feature value is a feature value that indicates the median value of luminance values in a defined range of a micrograph. That is, a median feature value is the result of taking a defined range "(number of pixels x) × (number of pixels y)" from each phase of a micrograph and taking a median luminance value within the range. Here, "number of pixels x" and "number of pixels y" may be the same size or different sizes. The area of "(number of pixels x) × (number of pixels y)" need not be rectangular, but is preferably a circular area when a micrograph is circular in shape, and may be calculated for a plurality of numbers of pixels x and y. Lower limits of "number of pixels x" and "number of pixels y" are preferably, for example, in a range that includes a size greater than noise in a micrograph and includes a size less than 1/2 the smaller crystal grain size of the plurality of phases of the metallic structure. When upper limits are too large, the mean feature value may be affected by grain boundaries or other adjacent phases, and therefore are preferably in a range that includes a size less than 1/2 the crystal grain size of the larger crystal grain size.

(C5: max feature value)

A max feature value is a feature value that indicates the maximum value of luminance values in a defined range of a micrograph. That is, a max feature value is the result of taking a defined range "(number of pixels x) × (number of pixels y)" from each phase of a micrograph and taking the maximum luminance value within the range. Here, "number of pixels x" and "number of pixels y" may be the same size or different sizes. The area of "(number of pixels x) × (number of pixels y)" need not be rectangular, but is preferably a circular area when a micrograph is circular in shape, and may be calculated for a plurality of numbers of pixels x and y. Lower limits of "number of pixels x" and "number of pixels y" are preferably, for example, in a range that includes a size greater than noise in a micrograph and includes a size less than 1/2 the smaller crystal grain size of the plurality of phases of the metallic structure. When upper limits are too large, the mean feature value may be affected by grain boundaries or other adjacent phases, and therefore are preferably in a range that includes a size less than 1/2 the crystal grain size of the larger crystal grain size.

(C6: min feature value)

A min feature value is a feature value that indicates the minimum value of luminance values in a defined range of a micrograph. That is, a min feature value is the result of taking a defined range "(number of pixels x) × (number of pixels y)" from each phase of a micrograph and taking the minimum luminance value within the range. Here, "number of pixels x" and "number of pixels y" may be the same size or different sizes. The area of "(number of pixels x) × (number of pixels y)" need not be rectangular, but is preferably a circular area when a micrograph is circular in shape, and may be calculated for a plurality of numbers of pixels x and y. Lower limits of "number of pixels x" and "number of pixels y" are preferably, for example, in a range that includes a size greater than noise in a micrograph and includes a size less than 1/2 the smaller crystal grain size of the plurality of phases of the metallic structure. When upper limits are too large, the mean feature value may be affected by grain boundaries or other adjacent phases, and therefore are preferably in a range that includes a size less than 1/2 the crystal grain size of the larger crystal grain size.

(C7: derivative feature value)

A derivative feature value is the result of taking a defined range "(number of pixels x) × (number of pixels y)" from each phase of a micrograph and calculating differential values in the x direction and the y direction relative to end pixels. Here, "number of pixels x" and "number of pixels y" may be the same size or different sizes. The area of "(number of pixels x) × (number of pixels y)" need not be rectangular, but is preferably a circular area when a micrograph is circular in shape, and may be calculated for a plurality of numbers of pixels x and y. Lower limits of "number of pixels x" and "number of pixels y" are preferably, for example, in a range that includes a size greater than noise in a micrograph and includes a size less than 1/2 the smaller crystal grain size of the plurality of phases of the metallic structure. When upper limits are too large, the mean feature value may be affected by grain boundaries or other adjacent phases, and therefore are preferably in a range that includes a size less than 1/2 the crystal grain size of the larger crystal grain size.

(C8: derivative sum feature value)

A derivative sum feature value is the convolution of a derivative feature value by calculating the mean feature value, the Gaussian feature value, the median feature value, the max feature value, and the min feature value for the derivative feature value described above. Here, "number of pixels x" and "number of pixels y" may be the same size or different sizes. The area of "(number of pixels x) × (number of pixels y)" need not be rectangular, but is preferably a circular area when a micrograph is circular in shape, and may be calculated for a plurality of numbers of pixels x and y. Lower limits of "number of pixels x" and "number of pixels y" are preferably, for example, in a range that includes a size greater than noise in a micrograph and includes a size less than 1/2 the smaller crystal grain size of the plurality of phases of the metallic structure. When upper limits are too large, the mean feature value may be affected by grain boundaries or other adjacent phases, and therefore are preferably in a range that includes a size less than 1/2 the crystal grain size of the larger crystal grain size.

Here, when an image is analyzed using an advanced image interpretation method, a boundary (outer circumferential portion) may be truncated for accurate analysis.

(Quantitative value calculation process)

The quantitative value calculation process is a process in which the quantitative value calculation unit 15 calculates quantitative values for the phases classified by the phase classification unit 14. For example, at least one of the following (1) to (6) is calculated as quantitative values.

In the specific examples described below, quantitative values (1) through (5) are calculated for each crystal grain of a specified phase after classification, and therefore multiple values are acquired even for a single micrograph. Further, aside from the specific examples described below, when another parameter can be used to quantify a micrograph, such a parameter may be used.

(1) Area fraction

Area fraction is calculated as the ratio of the area of each crystal grain of a specified phase to the area of the entire micrograph in each micrograph. Area fraction for each crystal grain is calculated according to the following expression.

[Math. 1]

$$f = \frac{A_i}{A_{sum}}$$

Here, f is area fraction for each crystal grain. $A_i$ is area for each crystal grain. $A_{sum}$ is area of the entire micrograph. Further, the area fraction for the specified phase per micrograph is calculated by calculating the sum of the area fraction of each crystal grain for the specified phase.

(2) Ellipsoid size

Ellipsoid size is calculated by approximating an ellipsoid to the shape of each crystal grain of the specified phase in each micrograph. The ellipsoid size is at least one of the major axis length, minor axis length, or aspect ratio of the approximated ellipsoid. Further, the average ellipsoid size for the specified phase per micrograph is calculated by calculating the average ellipsoid size of each crystal grain for the specified phase.

(3) Feret diameter

In each micrograph, after drawing a straight line from a boundary of each crystal grain of the specified phase, the Feret diameter with the greatest linear distance is calculated. Further, the average Feret diameter for the specified phase per micrograph is calculated by calculating the average Feret diameter calculated for each crystal grain.

(4) Average diameter

In each micrograph, the area of each crystal grain of the specified phase is determined and crystal grain average diameter is calculated by taking the square root of the area. Further, the average diameter for the specified phase per micrograph is calculated by calculating the average value from the average diameter calculated for each crystal grain for the specified phase.

(5) Circularity

In each micrograph, circularity of each crystal grain is calculated according to the following expression, by determining the area and circumference of each crystal grain of the specified phase.

[Math. 2]

$$C = 4\pi \times \frac{S}{p^2}$$

Here, C is circularity. S is area. p is circumference. When a crystal grain is a true circle, the circularity is 1.0. Conversely, the further the crystal grain shape is from a circle, the smaller the circularity becomes, below 1.0. Further, the average circularity for the specified phase per micrograph is calculated by calculating the average value from the circularity calculated for each crystal grain for the specified phase.

(6) Number density

In each micrograph, the number density per micrograph is calculated by counting the number of crystal grains of the specified phase and dividing the number of crystal grains by the area of the entire micrograph.

Preferably one or more of (1) to (6) are used as the quantitative values, and more preferably two or more are used. In particular, when determining representative magnification, the quantitative values preferably include the area fraction of (1).

(Magnification determination process)

The magnification determination process is a process in which the magnification determination unit 17 determines a representative magnification (step S14) based on the quantitative values calculated by the quantitative value calculation unit 15.

As described above, the quantitative values of crystal grains of the specified phase preferably include area fraction. The magnification determination unit 17 can determine the magnification as follows.

At a given magnification, when the number of micrographs in which there are two or more crystal grains each exceeding 10 % area fraction exceeds 1/3 of the total number of micrographs at the given magnification acquired in the input process, the magnification determination unit 17 adopts a smaller magnification. This is because a micrograph in which two or more crystal grains each exceed 10 % area fraction may result in missing grains at the boundaries (periphery) of the micrograph, or the size distribution of the grains may not be correctly evaluated due to the small number of grains imaged. Further, the number exceeding 1/3 of the total number means this is not a feature of some exceptional micrograph, but a trend in micrographs at the given magnification.

Further, at a given magnification, when the number of micrographs in which the crystal grain having the largest area fraction is less than 3 % of the total micrograph area exceeds 1/3 of the total number of micrographs at the given magnification acquired in the input process, the magnification determination unit 17 adopts a larger magnification. This is because a micrograph in which the crystal grain having the largest area fraction is less than 3 % of the total micrograph area has a large number of small crystal grains, and therefore crystal grain shape cannot be properly evaluated. Further, the number exceeding 1/3 of the total number means this is not a feature of some exceptional micrograph, but a trend in micrographs at the given magnification.

Hereinafter, for micrographs at a given magnification, a condition that no more than 1/3 of the total number of micrographs of the given magnification acquired in the input process contains two or more crystal grains each having an area fraction exceeding 10 % is also referred to as "Condition A". Further, for micrographs at a given magnification, a condition that no more than 1/3 of the total number of micrographs of the given magnification acquired in the input process are such that the crystal grain having the largest area fraction in a micrograph is less than 3 % of the total area of the micrograph is also referred to as "Condition B." In the magnification determination process, the magnification determination unit 17 sets a magnification that satisfies Condition A and Condition B as a representative magnification.

Here, the upper (10 %) and lower (3 %) limits of area fraction in Conditions A and B of the magnification determination process are examples, are not limited to these examples, and may be any values that allow appropriate evaluation of the size and shape of crystal grains of a target metal material. The quantitative values used in the conditions of the magnification determination process are area fraction in the present embodiment, but may be, for example, ellipsoid size, Feret diameter, or the like. Further, the quantitative values used in conditions of the magnification determination process may be, for example, circularity or number density, when the shape or number of crystal grains has a significant effect on material properties. According to the present embodiment, an example of using area fraction is described because the area fraction of a phase typically affects material properties in many cases.

(Deviation calculation process)

Here, it is possible that there are two or more levels of magnification that satisfy both Condition A and Condition B. When there is a plurality of candidates for representative magnification (Yes in step S15), the deviation calculation process is carried out according to the present embodiment (step S16). The deviation calculation process is a process in which the deviation calculation unit 16 calculates deviation of the quantitative values calculated by the quantitative value calculation unit 15.

The deviation calculation unit 16 calculates the standard deviation of quantitative values for each candidate representative magnification. The magnification with the smallest standard deviation is determined as the representative magnification. Here, when the order of standard deviation magnitude differs according to the quantitative value selected, the magnification with the smallest standard deviation of the quantitative value that is considered important is preferably determined as the representative magnification. For example, area fraction is considered to have the greatest effect on mechanical properties in DP steel sheets. Accordingly, the magnification with the smallest standard deviation for area fraction is preferably determined as the representative magnification. Further, calculating the deviation of all quantitative values calculated in the quantitative value calculation process is not necessary. For example, deviation need not be calculated for quantitative values that have little impact on the properties of a metal material.

When the deviation calculation process is executed, the magnification determination process is executed again. In the second magnification determination process, the magnification determination unit 17 determines one final representative magnification from the already selected candidate representative magnifications based on the deviation calculated by the deviation calculation unit 16.

(Output process)

When one representative magnification is determined (No in step S15), the output process is executed (step S17). The output process is a process in which the output interface 12 outputs the representative magnification determined.

In this way, the representative micrograph determining method executed by the representative micrograph determining device 10 according to the present embodiment determines an appropriate magnification when observing a metallic structure, independently of the subjectivity of an observer. Further, the conventional process of selection by an observer comparing micrographs while changing magnification can be eliminated, thereby improving work efficiency.

[Second embodiment]

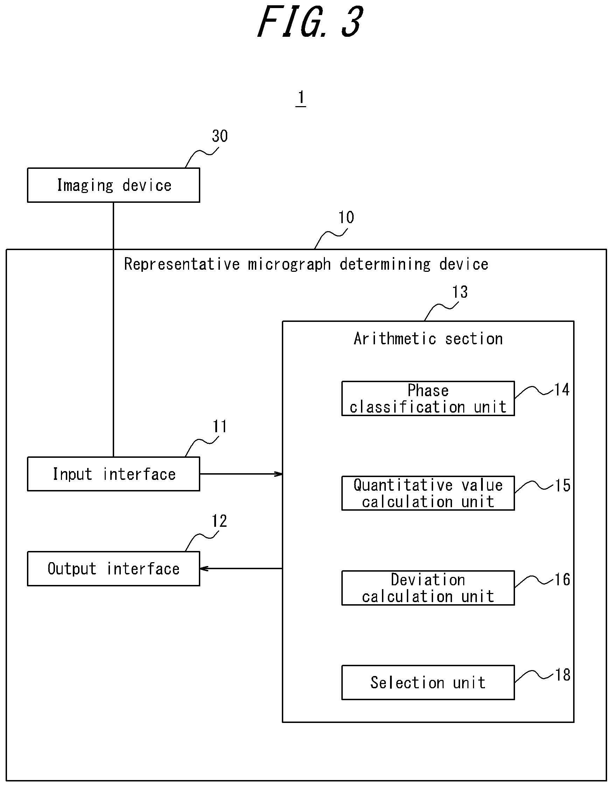

FIG. 3 is a block diagram of the representative micrograph determining system 1 including the representative micrograph determining device 10 according to a second embodiment. The representative micrograph determining device 10 according to the present embodiment selects a representative micrograph with an appropriate field of view when the representative magnification is known, for example. Compared to the representative micrograph determining device 10 according to the first embodiment, the representative micrograph determining device 10 according to the present embodiment includes many common components, but includes a selection unit 18 instead of the magnification determination unit 17. To avoid duplicate description, only configurations that differ from those of the first embodiment are described below.

(Selection unit)

The selection unit 18 selects a representative micrograph from a plurality of micrographs based on deviation of the quantitative values. The deviation of the quantitative values is calculated by the deviation calculation unit 16. According to the first embodiment, the deviation of the quantitative values is calculated when there are a plurality of candidates for representative magnification, but according to the present embodiment, the calculation of the deviation of the quantitative values is always executed. Further, according to the first embodiment, the standard deviation of the quantitative values was acquired for each magnification, but according to the second embodiment, the deviation is calculated for each field of view.

Further, according to the present embodiment, the input interface 11 acquires a plurality of micrographs taken at a defined magnification for two or more fields of view of a metal material. The defined magnification may be a known representative magnification, for example.

(Representative micrograph determining method)

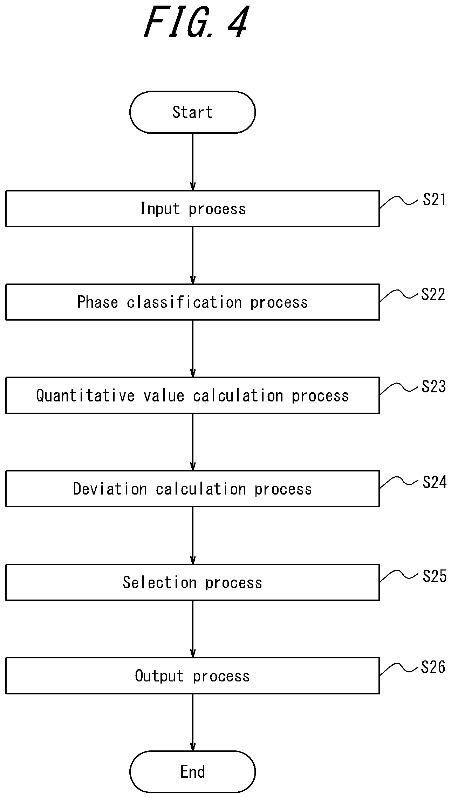

FIG. 4 is a flowchart illustrating processing of the representative micrograph determining method executed by the representative micrograph determining device 10 according to the present embodiment. In summary, the representative micrograph determining method includes, in this order: an input process of acquiring a plurality of micrographs taken at a defined magnification for two or more fields of view of a metal material; a phase classification process of classifying phases in the plurality of micrographs; a quantitative value calculation process of calculating quantitative values of the phases classified; a deviation calculation process of calculating deviation of the quantitative values; a selection process of selecting a representative micrograph from the plurality of micrographs based on the deviation; and an output process of outputting the representative micrograph. Here, when a representative micrograph could not be selected because the sum of deviations described below exceeds a defined value (0.5 as an example) or the like, the representative micrograph determining method may be executed again with an additional micrograph acquired in the input process.

The input process (step S21) is almost the same as the input process (step S11) of the first embodiment, but as described above, the input interface 11 acquires a plurality of micrographs taken at a defined magnification for two or more fields of view of a metal material.

The phase classification process (step S22) is the same as the phase classification process (step S12) according to the first embodiment. The quantitative value calculation process (step S23) is the same as the quantitative value calculation process (step S13) according to the first embodiment.

(Deviation calculation process)

The deviation calculation process (step S24) calculates deviation of the quantitative values as in the deviation calculation process (step S14) according to the first embodiment, but is always executed as described above and deviation is calculated for each field of view. The deviation calculation unit 16 calculates an average value $N_i$ of the quantitative values i of micrographs for all fields of view for the specified phase. Further, the deviation calculation unit 16 calculates an average value $\mu_{ij}$ of the quantitative values i of a micrograph for field of view j for the specified phase. The deviation calculation unit 16 calculates a normalized quantitative value i ($\mu_{ij}/N_i$) for field of view j by dividing the average value $\mu_{ij}$ of the quantitative values i of the micrograph for field of view j by the average value $N_i$ of the quantitative values i of the micrographs for all fields of view. Further, the deviation calculation unit 16 calculates the sum of deviations. The sum of deviations is calculated as the sum of "the absolute value of each normalized quantitative value i minus 1" and is represented by the expression below.

[Math. 3]

$$S_j = \sum_{i}^{n} \left| \frac{\mu_{ji}}{N_i} - 1 \right|$$

Here, $S_j$ is the sum of deviations for field of view j. n is the number of quantitative values employed.

Hereinafter, normalized quantitative value i is denoted by the prefix "normalized". For example, "normalized area fraction" means area fraction normalized by the deviation calculation process described above. Similarly, "normalized Feret diameter" and "normalized circularity" mean Feret diameter when normalized and circularity when normalized, respectively. Further, hereinafter, the deviation calculation process for each magnification as in the first embodiment may be referred to as the first deviation calculation process. Further, hereinafter, the deviation calculation process for each field of view as in the second embodiment may be referred to as the second deviation calculation process.

(Selection process)

The selection process (step S25) is a process in which the selection unit 18 selects a representative micrograph from a plurality of micrographs based on the deviation of quantitative values. The selection unit 18 first determines an appropriate field of view (representative field of view) based on the sum of deviations calculated by the deviation calculation unit 16, and selects a micrograph with the determined appropriate field of view as a representative micrograph. For example, a field of view having a relatively small sum of deviations is judged to be appropriate. Here, when there is a quantitative value that has a significant effect on material properties, then the selection unit 18 may specify a representative field of view based on such a quantitative value instead of the sum of deviations.

Once a representative micrograph is selected, the output process is executed (step S26). The output process is the process in which the output interface 12 outputs the representative micrograph selected.

In this way, the representative micrograph determining method executed by the representative micrograph determining device 10 according to the present embodiment selects a representative micrograph with an appropriate field of view for observing a metallic structure, independently of the subjectivity of an observer. Further, the conventional process of selection by an observer comparing a plurality of fields of view can be eliminated, thereby improving work efficiency.

[Third embodiment]

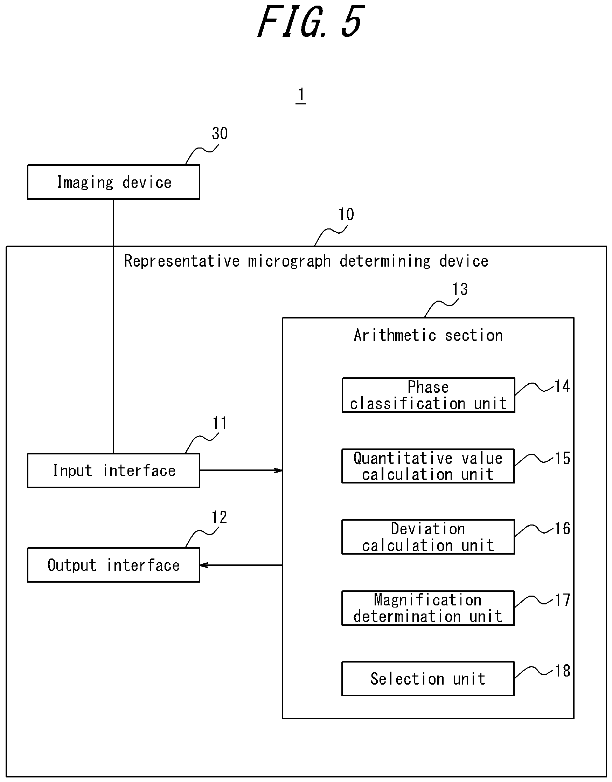

FIG. 5 is a block diagram of the representative micrograph determining system 1 including the representative micrograph determining device 10 according to a third embodiment. The representative micrograph determining device 10 according to the present embodiment determines a representative magnification and selects a representative micrograph with an appropriate field of view for the representative magnification determined. The representative micrograph determining device 10 according to the present embodiment includes components of the representative micrograph determining device 10 according to the first embodiment and the second embodiments, such as the selection unit 18 as well as the magnification determination unit 17. To avoid duplicate description, only configurations that differ from those of the first embodiment and the second embodiment are described below.

According to the present embodiment, as per the first embodiment, the input interface 11 acquires a plurality of micrographs taken at two or more magnifications for two or more fields of view of a metal material. The deviation calculation unit 16 and the selection unit 18 execute the second deviation calculation process and the selection process, respectively, with respect to a plurality of micrographs corresponding to a representative magnification determined by the magnification determination unit 17.

(Representative micrograph determining method)

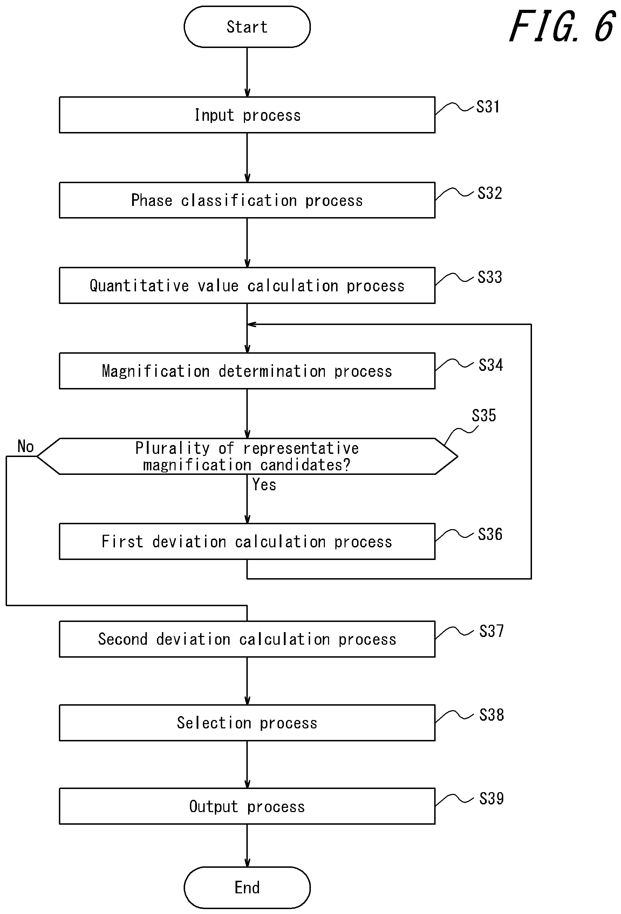

FIG. 6 is a flowchart illustrating processing of the representative micrograph determining method executed by the representative micrograph determining device 10 according to the present embodiment. In summary, the representative micrograph determining method includes, in this order: an input process of acquiring a plurality of micrographs taken at two or more magnifications for two or more fields of view of a metal material; a phase classification process of classifying phases in the plurality of micrographs; a quantitative value calculation process of calculating quantitative values of the phases classified; a magnification determination process of determining a representative magnification based on the quantitative values;

a deviation calculation process of calculating deviation of the quantitative values for a set of the plurality of micrographs corresponding to the representative magnification; a selection process of selecting a representative micrograph from the plurality of micrographs corresponding to the representative magnification based on the deviation; and an output process of outputting the representative micrograph. Here, when a representative micrograph could not be selected because the sum of deviations exceeds a defined value or the like, the representative micrograph determining method may be executed again with an additional micrograph acquired in the input process.

The input process (step S31), the phase classification process (step S32), the quantitative value calculation process (step S33), and the magnification determination process (step S34) are each the same as the process with the same name according to the first embodiment. Further, as in the first embodiment, when there are a plurality of candidates for a representative magnification (Yes in step S35), the first deviation calculation process is executed (step S36).

When one representative magnification is determined (No in step S35), the second deviation calculation process is executed (step S37). The second deviation calculation process is the same as that of the deviation calculation process (step S24) according to the second embodiment. The selection process (step S38) and the output process (step S39) are respectively the same as the processes with the same names according to the second embodiment.

In this way, the representative micrograph determining method executed by the representative micrograph determining device 10 according to the present embodiment selects a representative micrograph with an appropriate magnification and field of view for observing a metallic structure, independently of the subjectivity of an observer. Further, the conventional process of selection by an observer comparing a plurality of fields of view while changing magnification can be eliminated, thereby improving work efficiency.

(EXAMPLES)

Advantageous effects of the present disclosure are described in detail below based on examples, although the present disclosure is not limited to these examples.

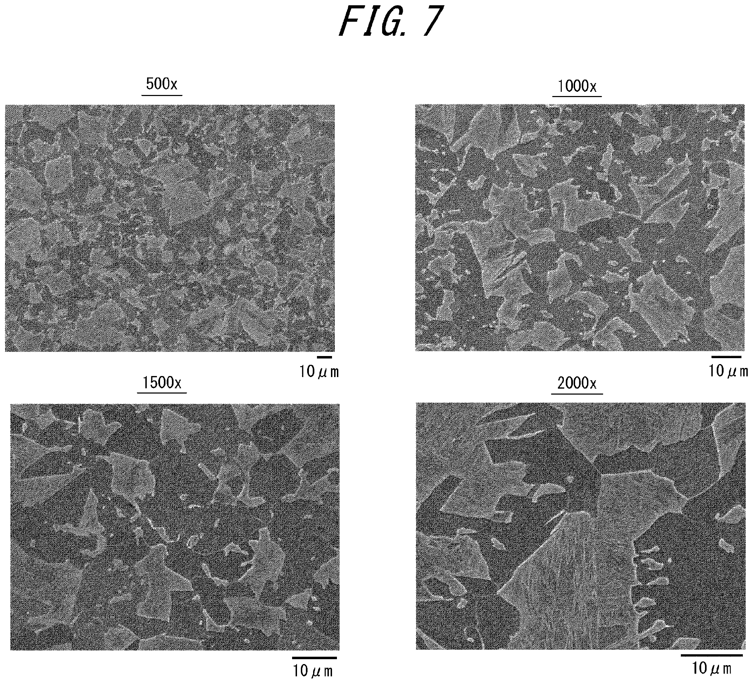

DP steel sheets consisting of ferrite phase and martensite phase were rough polished, and after finish polishing with diamond paste, the steel sheets were etched with nital. Micrographs were then taken for ten random fields of view using an electron microscope at magnifications of $500\times$, $1000\times$, $1500\times$, and $2000\times$. The plurality of micrographs taken were acquired (input process). FIG. 7 illustrates a portion of the acquired micrographs. Here, the phase indicated by white contrast is martensite and the phase indicated by black contrast is ferrite.

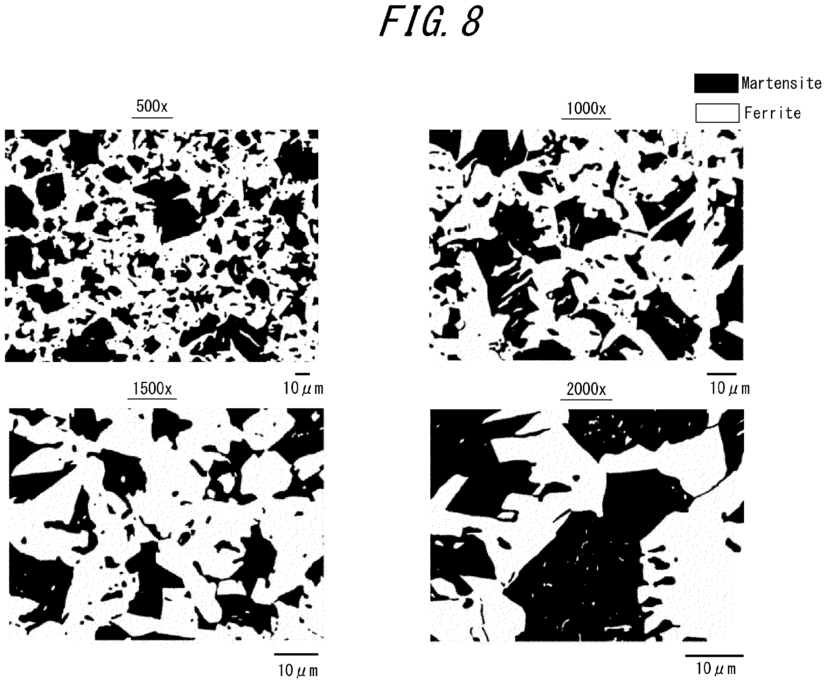

Next, the acquired micrographs were classified into ferrite phase and martensite phase as binary images using a random forest model, one of the advanced image interpretation methods described above (phase classification process). FIG. 8 illustrates the micrographs of FIG. 7 after the phase classification process. Here, black indicates martensite phase and white indicates ferrite phase.

Next, area fraction, Feret diameter, and circularity of each crystal grain were calculated as quantitative values for the martensite phase in each micrograph, based on the acquired binarized images (quantitative value calculation process).

Next, for the martensite phase in each micrograph, processing was executed to count the number of micrographs for each magnification in which two or more crystal grains each had an area fraction exceeding 10 %. Here, four or more fields of view corresponded to 1/3 of the number of micrographs at each magnification. Table 1 lists "+" for magnification that satisfied the conditions and "-" for magnification that did not. Here, in Table 1, Condition A is that no more than 1/3 of the total number of micrographs of the given magnification acquired in the input process contains two or more crystal grains each having an area fraction exceeding 10 %. Further, Condition B is that no more than 1/3 of the total number of micrographs of the given magnification acquired in the input process are such that the crystal grain having the largest area fraction in a micrograph is less than 3 % of the total micrograph area.

[Table 1]

(Table 1)

| Magnification [times] | Condition A | Condition B | Area fraction [%] | | Feret diameter [μm] | | Circularity [-] | |
|---|---|---|---|---|---|---|---|---|
| | | | Average | Standard deviation | Average | Standard deviation | Average | Standard deviation |
| 500 | + | + | 43 | 2.43 | 9.45 | 0.55 | 0.67 | 0.013 |
| 1000 | + | + | 48 | 8 | 6.19 | 0.86 | 0.69 | 0.031 |
| 1500 | + | + | 37 | 7 | 5.56 | 0.81 | 0.63 | 0.035 |
| 2000 | - | + | 35 | 7 | 4.79 | 0.84 | 0.61 | 0.045 |

As indicated in the Condition A column of Table 1, the magnification at $2000\times$ was determined to be inappropriate, and the magnifications at $500\times$, $1000\times$, and $1500\times$ were determined to be appropriate. For example, in FIG. 8, the micrograph taken at $2000\times$ confirmed that the size of the martensite phase could not be properly evaluated because all the martensite phase reached the boundaries of the image.

For the martensite phase of each micrograph, processing was executed to count the number of micrographs for each magnification in which the crystal grain having the largest area fraction in the micrograph is less than 3 % of the total micrograph. Here, four or more fields of view corresponded to 1/3 of the number of micrographs at each magnification. Table 1 lists "+" for magnification that satisfied the conditions and "-" for magnification that did not. As indicated in the Condition B column of Table 1, the magnifications at $500\times$, $1000\times$, and $1500\times$ were all determined to be appropriate.

Average and standard deviation were calculated for each quantitative value of each micrograph for each magnification (first deviation calculation process). As indicated in Table 1, the standard deviation of each quantitative value for each magnification was found to be the smallest when images were taken at $500\times$. From this result, $500\times$ was determined as a representative magnification (magnification determination process).

After the representative magnification was determined, normalization was performed on the quantitative values for each micrograph taken at the representative magnification, and the sum of the deviations was calculated (second deviation calculation process). Table 2 indicates the values of the calculation for each field of view. The micrograph for the 5th field of view was selected as a representative micrograph because the sum of deviations for the 5th field of view was the smallest (selection process). Here, the Feret diameter in Table 2 indicates the average Feret diameter per micrograph.

[Table 2]

(Table 2)

| Field of view (ID No.) | Area fraction [%] | Normalized area fraction [-] | Feret diameter [μm] | Normalized Feret diameter [-] | Circularity [-] | Normalized circularity [-] | Sum of deviation [-] |
|---|---|---|---|---|---|---|---|
| 1 | 49 | 1.13 | 8.9 | 0.94 | 0.69 | 1.03 | 0.22 |
| 2 | 41 | 0.94 | 9.6 | 1.01 | 0.67 | 1.00 | 0.07 |
| 3 | 42 | 0.98 | 8.7 | 0.92 | 0.69 | 1.02 | 0.12 |
| 4 | 44 | 1.03 | 9.2 | 0.98 | 0.68 | 1.01 | 0.06 |

(continued)

| Field of view (ID No.) | Area fraction [%] | Normalized area fraction [-] | Feret diameter [μm] | Normalized Feret diameter [-] | Circularity [-] | Normalized circularity [-] | Sum of deviation [-] |
|---|---|---|---|---|---|---|---|
| 5 | 43 | 0.99 | 9.4 | 0.99 | 0.66 | 0.99 | 0.03 |
| 6 | 45 | 1.04 | 9.9 | 1.05 | 0.64 | 0.96 | 0.13 |
| 7 | 41 | 0.95 | 9.4 | 1.00 | 0.68 | 1.01 | 0.06 |
| 8 | 40 | 0.94 | 9.8 | 1.03 | 0.66 | 0.99 | 0.10 |
| 9 | 42 | 0.97 | 9.1 | 0.96 | 0.68 | 1.01 | 0.08 |
| 10 | 44 | 1.03 | 10.6 | 1.12 | 0.66 | 0.99 | 0.16 |

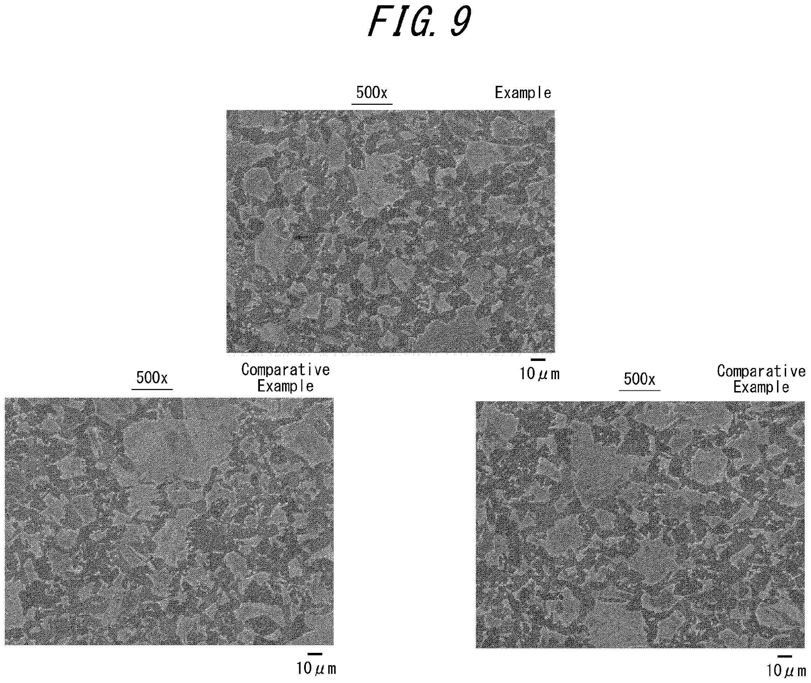

The representative micrograph determined by the present disclosure is illustrated as an Example in FIG. 9. The two Comparative Examples in FIG. 9 are micrographs that were not determined to be representative micrographs.

The representative micrograph has no relatively large martensite crystal grains and the like, and can be said to have an average microstructure, indicating that the micrograph is appropriate as a representative micrograph. In contrast, the Comparative Examples have relatively large martensite crystal grains and the like.

This, according to the present Example, a representative micrograph of a metal material could be determined objectively without arbitrariness. As a result, correlation with material properties can be objectively considered, and efficient material development can be expected. Further, quantitative values of each phase can be evaluated appropriately in the evaluation of steel material or metal material with multiple metallic phases, and therefore development of efficient steel material or metal material with multiple metallic phases may be expected.

As described above, the representative micrograph determining method, the representative micrograph determining device, the imaging device, and the program can objectively and efficiently determine a representative micrograph from a plurality of micrographs of a metal material according to the configurations and processes described above.

Although embodiments of the present disclosure have been described based on the drawings and examples, it should be noted that a person skilled in the art may make variations and modifications based on the present disclosure. Therefore, it should be noted that such variations and modifications are included within the scope of the present disclosure. For example, functions and the like included in each component and step may be rearranged, and a plurality of components and steps may be combined into one or divided, as long as no logical inconsistency results. The embodiments according to the present disclosure may be realized as a storage medium on which a program executed by a processor provided to a device is stored. The scope of the present disclosure should be understood to include these examples.

REFERENCE SIGNS LIST

1       representative micrograph determining system
10      representative micrograph determining device
11      input interface
12      output interface
13      arithmetic section
14      phase classification unit
15      quantitative value calculation unit
16      deviation calculation unit
17      magnification determination unit
18      selection unit
30      imaging device

**Claims**

1.   A representative micrograph determining method comprising:

an input process of acquiring a plurality of micrographs taken at two or more magnifications for two or more fields of view of a metal material;
a phase classification process of classifying phases in the plurality of micrographs;
a quantitative value calculation process of calculating quantitative values of the phases classified;
a magnification determination process of determining a representative magnification based on the quantitative values; and
an output process of outputting the representative magnification.

2. A representative micrograph determining method comprising:

an input process of acquiring a plurality of micrographs taken at a defined magnification for two or more fields of view of a metal material;
a phase classification process of classifying phases in the plurality of micrographs;
a quantitative value calculation process of calculating quantitative values of the phases classified;
a deviation calculation process of calculating deviation of the quantitative values;
a selection process of selecting a representative micrograph from the plurality of micrographs based on the deviation; and
an output process of outputting the representative micrograph.

3. A representative micrograph determining method comprising:

an input process of acquiring a plurality of micrographs taken at two or more magnifications for two or more fields of view of a metal material;
a phase classification process of classifying phases in the plurality of micrographs;
a quantitative value calculation process of calculating quantitative values of the phases classified;
a magnification determination process of determining a representative magnification based on the quantitative values; and
a deviation calculation process of calculating deviation of the quantitative values for a set of the plurality of micrographs corresponding to the representative magnification;
a selection process of selecting a representative micrograph from the set of the plurality of micrographs corresponding to the representative magnification based on the deviation; and
an output process of outputting the representative micrograph.

4. The representative micrograph determining method according to any one of claims 1 to 3, wherein the quantitative values are at least one of area fraction, ellipsoid size, Feret diameter, average diameter, circularity, or number density.

5. A representative micrograph determining device comprising:

an input interface configured to acquire a plurality of micrographs taken at two or more magnifications for two or more fields of view of a metal material;
a phase classification unit configured to classify phases in the plurality of micrographs;
a quantitative value calculation unit configured to calculate quantitative values of the phases classified;
a magnification determination unit configured to determine a representative magnification based on the quantitative values; and
an output interface configured to output the representative magnification.

6. A representative micrograph determining device comprising:

an input interface configured to acquire a plurality of micrographs taken at a defined magnification for two or more fields of view of a metal material;
a phase classification unit configured to classify phases in the plurality of micrographs;
a quantitative value calculation unit configured to calculate quantitative values of the phases classified;
a deviation calculation unit configured to calculate deviation of the quantitative values;
a selection unit configured to select a representative micrograph from the plurality of micrographs based on the deviation; and
an output interface configured to output the representative micrograph.

7. A representative micrograph determining device comprising:

an input interface configured to acquire a plurality of micrographs taken at two or more magnifications for two or more fields of view of a metal material;

a phase classification unit configured to classify phases in the plurality of micrographs;

a quantitative value calculation unit configured to calculate quantitative values of the phases classified;

a magnification determination unit configured to determine a representative magnification based on the quantitative values; and

a deviation calculation unit configured to calculate deviation of the quantitative values for a set of the plurality of micrographs corresponding to the representative magnification;

a selection unit configured to select a representative micrograph from the set of the plurality of micrographs corresponding to the representative magnification based on the deviation; and

an output interface configured to output the representative micrograph.

8. An imaging device configured to take the plurality of micrographs acquired by the representative micrograph determining device according to any one of claims 5 to 7.

9. The imaging device according to claim 8, wherein the imaging device is an optical microscope or a scanning electron microscope.

10. A program configured to

cause a computer to function as

an input interface configured to acquire a plurality of micrographs taken at two or more magnifications for two or more fields of view of a metal material;

a phase classification unit configured to classify phases in the plurality of micrographs;

a quantitative value calculation unit configured to calculate quantitative values of the phases classified;

a magnification determination unit configured to determine a representative magnification based on the quantitative values; and

an output interface configured to output the representative magnification.

11. A program configured to

cause a computer to function as

an input interface configured to acquire a plurality of micrographs taken at a defined magnification for two or more fields of view of a metal material;

a phase classification unit configured to classify phases in the plurality of micrographs;

a quantitative value calculation unit configured to calculate quantitative values of the phases classified;

a deviation calculation unit configured to calculate deviation of the quantitative values;

a selection unit configured to select a representative micrograph from the plurality of micrographs based on the deviation; and

an output interface configured to output the representative micrograph.

12. A program configured to

cause a computer to function as

an input interface configured to acquire a plurality of micrographs taken at two or more magnifications for two or more fields of view of a metal material;

a phase classification unit configured to classify phases in the plurality of micrographs;

a quantitative value calculation unit configured to calculate quantitative values of the phases classified;

a magnification determination unit configured to determine a representative magnification based on the quantitative values; and

a deviation calculation unit configured to calculate deviation of the quantitative values for a set of the plurality of micrographs corresponding to the representative magnification;

a selection unit configured to select a representative micrograph from the set of the plurality of micrographs corresponding to the representative magnification based on the deviation; and

an output interface configured to output the representative micrograph.

# FIG. 1

<u>1</u>

Imaging device — 30

Representative micrograph determining device — 10

Arithmetic section — 13

Phase classification unit — 14

Input interface — 11

Quantitative value calculation unit — 15

Output interface — 12

Deviation calculation unit — 16

Magnification determination unit — 17

# FIG. 2

```
                        ┌──────────┐
                        │  Start   │
                        └──────────┘
                             │
                             ▼
         ┌─────────────────────────────────────┐
         │           Input process             │──── S11
         └─────────────────────────────────────┘
                             │
                             ▼
         ┌─────────────────────────────────────┐
         │      Phase classification process   │──── S12
         └─────────────────────────────────────┘
                             │
                             ▼
         ┌─────────────────────────────────────┐
         │  Quantitative value calculation process │──── S13
         └─────────────────────────────────────┘
                             │
                             ▼
         ┌─────────────────────────────────────┐
         │   Magnification determination process│──── S14
         └─────────────────────────────────────┘
                             │
                             ▼                      S15
       No  ╱─────────────────────────────────────╲
      ◄────   Plurality of representative
              magnification candidates?
           ╲─────────────────────────────────────╱
                         │ Yes
                         ▼
         ┌─────────────────────────────────────┐
         │     Deviation calculation process   │──── S16
         └─────────────────────────────────────┘

                             │
                             ▼
         ┌─────────────────────────────────────┐
         │           Output process            │──── S17
         └─────────────────────────────────────┘
                             │
                             ▼
                        ┌──────────┐
                        │   End    │
                        └──────────┘
```

# FIG. 3

1

30

Imaging device

10

Representative micrograph determining device

13

Arithmetic section

Phase classification unit  14

11

Input interface

Quantitative value calculation unit  15

12

Output interface

Deviation calculation unit  16

Selection unit  18

# FIG. 4

```
        ┌─────────────┐
        │    Start    │
        └──────┬──────┘
               │
               ▼
┌──────────────────────────────┐
│         Input process        │ ～ S21
└──────────────┬───────────────┘
               │
               ▼
┌──────────────────────────────┐
│   Phase classification process   │ ～ S22
└──────────────┬───────────────┘
               │
               ▼
┌──────────────────────────────┐
│ Quantitative value calculation process │ ～ S23
└──────────────┬───────────────┘
               │
               ▼
┌──────────────────────────────┐
│   Deviation calculation process   │ ～ S24
└──────────────┬───────────────┘
               │
               ▼
┌──────────────────────────────┐
│       Selection process      │ ～ S25
└──────────────┬───────────────┘
               │
               ▼
┌──────────────────────────────┐
│        Output process        │ ～ S26
└──────────────┬───────────────┘
               │
               ▼
        ┌─────────────┐
        │     End     │
        └─────────────┘
```

# *FIG. 5*

<u>1</u>

```
                30
              ┌──────────────────┐
              │  Imaging device  │
              └────────┬─────────┘
                       │
                                        10
┌──────────────────────┼──────────────────────────────────────────────┐
│                       Representative micrograph determining device    │
│                       │                         13                    │
│                       │              ┌────────────────────────────────┐│
│                       │              │      Arithmetic section        ││
│                       │              │                                ││
│                       │              │  ┌──────────────────┐    14    ││
│                       │              │  │      Phase       │          ││
│                       │              │  │ classification   │          ││
│                       │              │  │      unit        │          ││
│                       │              │  └──────────────────┘          ││
│              11       │              │                                ││
│     ┌──────────────────┐            │  ┌──────────────────┐    15    ││
│     │                  │            │  │ Quantitative value│          ││
│     │ Input interface  ├───────────▶│  │ calculation unit  │          ││
│     │                  │            │  └──────────────────┘          ││
│     └──────────────────┘            │                                ││
│              12                     │  ┌──────────────────┐    16    ││
│     ┌──────────────────┐            │  │    Deviation      │          ││
│     │                  │            │  │ calculation unit  │          ││
│     │ Output interface │◀───────────│  └──────────────────┘          ││
│     │                  │            │                                ││
│     └──────────────────┘            │  ┌──────────────────┐    17    ││
│                                     │  │  Magnification    │          ││
│                                     │  │ determination unit│          ││
│                                     │  └──────────────────┘          ││
│                                     │                                ││
│                                     │  ┌──────────────────┐    18    ││
│                                     │  │  Selection unit   │          ││
│                                     │  └──────────────────┘          ││
│                                     └────────────────────────────────┘│
└───────────────────────────────────────────────────────────────────────┘
```

# FIG. 6

Start

Input process — S31

Phase classification process — S32

Quantitative value calculation process — S33

Magnification determination process — S34

S35
Plurality of representative magnification candidates?
No / Yes

First deviation calculation process — S36

Second deviation calculation process — S37

Selection process — S38

Output process — S39

End

## FIG. 7

# FIG. 8

■ Martensite
□ Ferrite

500x

1000x

10 μm

1500x

2000x

10 μm

10 μm

10 μm

FIG. 9

## INTERNATIONAL SEARCH REPORT

| International application No. |
|---|
| **PCT/JP2023/013409** |

| A. | CLASSIFICATION OF SUBJECT MATTER |
|---|---|

*G01N 33/2028*(2019.01)i; *G01N 21/17*(2006.01)i
FI:   G01N33/2028; G01N21/17 A

According to International Patent Classification (IPC) or to both national classification and IPC

| B. | FIELDS SEARCHED |
|---|---|

Minimum documentation searched (classification system followed by classification symbols)

G01N33/2028; G01N21/17

Documentation searched other than minimum documentation to the extent that such documents are included in the fields searched

Published examined utility model applications of Japan 1922-1996
Published unexamined utility model applications of Japan 1971-2023
Registered utility model specifications of Japan 1996-2023
Published registered utility model applications of Japan 1994-2023

Electronic data base consulted during the international search (name of data base and, where practicable, search terms used)

| C. | DOCUMENTS CONSIDERED TO BE RELEVANT |
|---|---|

| Category* | Citation of document, with indication, where appropriate, of the relevant passages | Relevant to claim No. |
|---|---|---|
| X | WO 2021/199937 A1 (JFE STEEL CORP) 07 October 2021 (2021-10-07)<br>entire text, all drawings, in particular, see paragraphs [0014]-[0024], [0082]-[0090], fig. 2, 5, etc. | 1, 4-5, 8-10 |
| A | WO 2021/153633 A1 (JFE STEEL CORP) 05 August 2021 (2021-08-05)<br>entire text, all drawings | 1-12 |
| A | WO 2022/059186 A1 (TOSHIBA KK) 24 March 2022 (2022-03-24)<br>see entire text, all drawings, etc. | 1-12 |
| A | JP 2019-070738 A (OLYMPUS CORP) 09 May 2019 (2019-05-09)<br>see entire text, all drawings, etc. | 1-12 |
| A | JP 2012-008307 A (OLYMPUS IMAGING CORP) 12 January 2012 (2012-01-12)<br>see entire text, all drawings, etc. | 1-12 |
| A | JP 07-318787 A (CANON INC) 08 December 1995 (1995-12-08)<br>see entire text, all drawings, etc. | 1-12 |

☐ Further documents are listed in the continuation of Box C.     ☑ See patent family annex.

| * | Special categories of cited documents: |
|---|---|
| "A" | document defining the general state of the art which is not considered to be of particular relevance |
| "E" | earlier application or patent but published on or after the international filing date |
| "L" | document which may throw doubts on priority claim(s) or which is cited to establish the publication date of another citation or other special reason (as specified) |
| "O" | document referring to an oral disclosure, use, exhibition or other means |
| "P" | document published prior to the international filing date but later than the priority date claimed |

| "T" | later document published after the international filing date or priority date and not in conflict with the application but cited to understand the principle or theory underlying the invention |
|---|---|
| "X" | document of particular relevance; the claimed invention cannot be considered novel or cannot be considered to involve an inventive step when the document is taken alone |
| "Y" | document of particular relevance; the claimed invention cannot be considered to involve an inventive step when the document is combined with one or more other such documents, such combination being obvious to a person skilled in the art |
| "&" | document member of the same patent family |

| Date of the actual completion of the international search | Date of mailing of the international search report |
|---|---|
| **08 June 2023** | **20 June 2023** |

| Name and mailing address of the ISA/JP | Authorized officer |
|---|---|
| **Japan Patent Office (ISA/JP)**<br>**3-4-3 Kasumigaseki, Chiyoda-ku, Tokyo 100-8915**<br>**Japan** | |
| | Telephone No. |

Form PCT/ISA/210 (second sheet) (January 2015)

**INTERNATIONAL SEARCH REPORT**
**Information on patent family members**

International application No.

**PCT/JP2023/013409**

| Patent document cited in search report | | | Publication date (day/month/year) | Patent family member(s) | | | Publication date (day/month/year) |
|---|---|---|---|---|---|---|---|
| WO | 2021/199937 | A1 | 07 October 2021 | EP | 4131159 | A1 | |
| | | | | entire text, all drawings, in particular, claims, see paragraphs [0014]-[0024], [0082]-[0090], fig. 2, 5, etc. | | | |
| | | | | CN | 115398228 | A | |
| | | | | KR | 10-2022-0156044 | A | |
| WO | 2021/153633 | A1 | 05 August 2021 | EP | 4099012 | A1 | |
| | | | | see entire text, all drawings, etc. | | | |
| | | | | KR | 10-2022-0117328 | A | |
| | | | | CN | 115038965 | A | |
| WO | 2022/059186 | A1 | 24 March 2022 | US | 2022/0318983 | A1 | |
| | | | | see entire text, all drawings, etc. | | | |
| JP | 2019-070738 | A | 09 May 2019 | (Family: none) | | | |
| JP | 2012-008307 | A | 12 January 2012 | (Family: none) | | | |
| JP | 07-318787 | A | 08 December 1995 | (Family: none) | | | |

Form PCT/ISA/210 (patent family annex) (January 2015)

**EP 4 492 056 A1**

**Patent documents cited in the description**

- JP 2009287941 A